# EUROPÄISCHE PATENTSCHRIFT

(11) **EP 0 688 534 B1**
(45) Veröffentlichungstag und Bekanntmachung des Hinweises auf die Patenterteilung: **12.11.1997**
(21) Anmeldenummer: 95106955.8
(22) Anmeldetag: 09.05.1995
(51) Int. Cl.: A61B 17/28

(54) **Chirurgisches Rohrschaftinstrument**
Tubular shaft surgical instrument
Instrument chirurgical à tige tubulaire

(30) Priorität: 21.06.1994 DE 4421585; 18.01.1995 DE 29500698 U
(43) Veröffentlichungstag der Anmeldung: 27.12.1995
(73) Patentinhaber: AESCULAP AG, 78532 Tuttlingen (DE)
(72) Erfinder: Morales, Pedro, D-78532 Tuttlingen (DE); Wäschle, Andreas, D-78598 Königsheim (DE); Weisshaupt, Dieter, D-78194 Immendingen (DE); Zepf, Rudolf, D-78604 Rietheim-Weilheim (DE); Psalmon, François, F-23000 Gueret (FR)
(74) Vertreter: Hoeger, Stellrecht & Partner

(56) Entgegenhaltungen:
- EP-A- 0 535 370

## Beschreibung

Die Erfindung betrifft ein chirurgisches Rohrschaftinstrument mit einem Schaft und einer darin verschieblich gelagerten Schub- und Zugstange zur Bewegung eines Werkzeugs am Ende des Schafts und mit einem koaxial zum Schaft angeordneten Griffteil am anderen Ende des Schafts, in dem ein Handgriff um eine quer zur Schaftlängsachse verlaufende Drehachse verschwenkbar gelagert ist, dessen Bewegung über Getriebemittel auf die Schub- und Zugstange übertragbar ist, und mit einem federnden Rastelement, das bei der Bewegung des Handgriffs von einer Ausgangsstellung aus an einer Raste vorbeibewegt wird, dabei in diese eingreift und dadurch eine Rückbewegung des Handgriffs verhindert.

Ein solches chirurgisches Rohrschaftinstrument ist beispielsweise aus der EP 0535370 A1 bekannt. Es ist mit einem solchen Instrument möglich, ein Werkzeug in einer bestimmten Stellung zu fixieren, beispielsweise ein Zangenwerkzeug in einer bestimmten Schließposition. Um diese Schließposition wieder aufzugeben und das Werkzeug wieder zu öffnen, ist es bei dem bekannten Rohrschaftinstrument notwendig, mit der zweiten Hand ein spezielles Arretierelement am Griffteil zu betätigen, dadurch wird das federnde Rastelement aus der Raste ausgehoben. Um in dieser Weise die Raste lösen zu können, benötigt der Operateur seine zweite Hand, und dies in vielen Fällen hinderlich.

Bei zangenartigen chirurgischen Instrumenten mit zwei gegeneinander verschwenkbaren Handgriffen ist eine Griffsperre bekannt, bei welcher ein Sperrenteil eine Kulissenbahn und das zweite Sperrenteil einen in die Kulissenbahn eingreifenden Kulissenzapfen aufweist, die derart zusammenwirken, daß eine Sperre der beiden Instrumententeile durch vollständiges Schließen des Instrumentes aufgehoben werden kann (DE 42 07 124 C1). Dies setzt das komplizierte Ineinandergreifen der Kulissenbahn und des Kulissenzapfens voraus und beschränkt sich auf Instrumente mit gegeneinander verschwenkbaren Branchen.

Es ist Aufgabe der Erfindung ein chirurgisches Rohrschaftinstrument der eingangs beschriebenen Art so auszubilden, daß ein Lösen der Rastverbindung möglich ist, ohne daß der Operateur eine zweite Hand benötigt oder das Instrument in irgendeiner Weise in der Hand umsetzen muß.

Diese Aufgabe wird bei einem chirurgischen Rohrschaftinstrument der eingangs beschriebenen Art erfindungsgemäß dadurch gelöst, daß das Rastelement und die Raste eine Umlaufsperre bilden, bei der das Rastelement am Ende des Hubs des Handgriffs an der Raste vollständig vorbeibewegt ist und bei der Rückbewegung an der Rückseite der Raste vorbei in die Ausgangsstellung gelangt.

Es ist für den Operateur dadurch möglich, die Rastverbindung in einfacher Weise wieder zu lösen. Es genügt dazu nämlich, den Handgriff vollständig in die eine Endposition zu verschwenken, in der der Handgriff am weitesten in das Griffteil eingeschwenkt ist, dabei gleitet das Rastelement federnd an der gesamten Raste vorbei. Bei der Rückbewegung wird das federnde Rastelement an der Rückseite der Raste vorbeigelenkt, dies erfolgt durch geeignete Umlenkflächen an der Raste und/oder dem Rastelement, so daß der Handgriff wieder in die Offenstellung zurückgelangen kann. Beim erneuten Schließen wird das Rastelement wieder durch geeignete Umlenkflächen an der Raste vorbeigeführt und kann daher in diese einrasten und den Handgriff in einer bestimmten Position fixieren.

Günstig ist es dabei, wenn die Raste am Handgriff gehalten ist, insbesondere, wenn die Raste am freien Ende des Handgriffs senkrecht zur Längsausdehnung des Handgriffs angeordnet ist.

Es kann dann insbesondere vorgesehen sein, daß das federnde Rastelement im Griffteil im wesentlichen parallel zum Schaft verschieblich gelagert ist.

Eine weitere bevorzugte Ausgestaltung ergibt sich dann, wenn das Rastelement mittels eines Betätigungselements zwischen zwei Stellungen verschiebbar ist, nämlich einer vorgeschobenen Rastposition, in der das Rastelement mit der Raste als Umlaufsperre wirkt, und einer zurückgezogenen Freilaufposition, in der das Rastelement von der Raste dauerhaft entfernt ist. Der Operateur kann daher vor dem Schließen des Werkzeugs entscheiden, ob er die Rastfunktion verwenden will oder nicht, entsprechend wird das Rastelement vor dem Schließen durch Betätigung des Betätigungselements eingestellt.

Um das Umlaufen des Rastelements um die Raste zu ermöglichen, ist es vorteilhaft, wenn das federnde Rastelement am Griffteil von einer Mittelstellung aus in beiden Bewegungsrichtungen gegen die Wirkung einer Feder elastisch verschiebbar ist.

Dabei ist es vorteilhaft, wenn die Federkraft, durch die das Rastelement in die Raste gedrückt wird, größer ist als die Federkraft, die das Rastelement gegen die Rückseite der Raste andrückt. Dadurch wird erreicht, daß das Rastelement mit einer großen Federkraft in die Raste gedrückt wird, so daß eine sichere Rastverbindung entsteht. Andererseits genügt es für die Rückführung des Rastelements in die Ausgangsstellung, wenn das Rastelement mit einer geringen Kraft verschoben wird. Die Verwendung einer schwächeren Feder ermöglicht hier eine Bewegung des Handgriffs mit geringem Kraftaufwand und mit geringerer Abnützung.

Bei einer bevorzugten Ausführungsform ist vorgesehen, daß das Rastelement an einem Kolben gehalten ist, der von einer ersten Feder beaufschlagt bis an einen Anschlag in einen Hohlkolben einschiebbar ist, und daß der Hohlkolben von einer zweiten Feder in entgegengesetzter Richtung gegen einen festen Anschlag einer Führung des Hohlkolbens beaufschlagt wird. Damit ergibt sich eine Verschiebung des Rastelements entgegen der ersten Feder, wenn der Kolben aus dem Hohlkolben ausgeschoben wird, und eine Verschiebung des Rastelements in der entgegengesetzten Richtung, wenn der Kolben an dem Anschlag des Hohlkolbens anliegend und diesen daher mitnehmend in der entgegengesetzten Richtung verschoben wird. Im ersten Fall wird die erste Feder wirksam, im zweiten Fall die zweite, die gegebenenfalls eine wesentlich höhere Federkraft haben kann.

Es ist dabei besonders vorteilhaft, wenn gemäß einer bevorzugten Weiterbildung der Erfindung die Führung eine Zylinderhülse ist, die mittels des Betätigungselements im Griffteil verschiebbar ist. Durch die Verschiebung der Zylinderhülse läßt sich nämlich dann das federnde Rastelement wahlweise in die für die Umlaufsperre wirksame Position oder in die unwirksame Freilaufposition verschieben. Dabei ist es vorteilhaft, wenn die Zylinderhülse in einer Bohrung des Griffteils verschiebbar ist, insbesondere wenn die Zylinderhülse in der Bohrung in den beiden Endstellungen durch elastische Rasten gehalten ist. Durch geeignete Abstimmungen der Federkraft dieser elastischen Rasten einerseits und der Feder, die das elastische Rastelement verschiebt andererseits, kann beispielsweise erreicht werden, daß dieses bei der Betätigung der Umlaufsperre und bei dem Rücklauf des Handgriffs selbsttätig in die Freilaufposition verschoben wird, nach jeder vollständigen Betätigung des Handgriffs muß der Operateur daher erneut das elastische Rastelement in die Rastposition verschieben, wenn er die Funktion der Umlaufsperre ausnützen will. Dadurch läßt sich sicherstellen, daß nicht ungewollt die Umlaufsperrfunktion eingeschaltet wird.

Weiterhin ist gemäß einer bevorzugten Ausführungsform vorgesehen, daß die Getriebemittel eine Federverbindung umfassen, die nach dem Erreichen einer Endposition der Schub- und Zugstange eine weitere Schließbewegung des Handgriffs ermöglichen. Beim Einschwenken des Handgriffs wird also zunächst eine Endposition der Schub- und Zugstange erreicht, darüber hinaus ist aber eine weitere VerSchwenkung des Handgriffs möglich, ohne daß die Schub- und Zugstange weiterbewegt wird, da eine Federverbindung hier einen Überhub des Handgriffs ermöglicht. Dadurch läßt sich die Umlaufsperre lösen, ohne daß dabei die Schub- und Zugstange weiterverschoben wird. Damit läßt sich insbesondere eine Überbelastung der Werkzeuge vermeiden.

Günstig ist es, wenn die Endposition der Schub- und Zugstange durch einen Anschlag definiert wird, an dem ein Teil der Schub- und Zugstange zur Anlage kommt.

Bei einer bevorzugten Ausführungsform kann vorgesehen sein, daß die Federverbindung durch eine die Schub- und Zugstange umgebende sich einseitig an einem Vorsprung derselben abstützende Druckfeder gebildet wird, an deren anderem Ende eine die Druckfeder umgebende Zughülse angreift.

Bei einer weiteren bevorzugten Ausführungsform ist vorgesehen, daß die Teile der Umlaufsperre erst in Eingriff gelangen, wenn das Betätigungselement eine Position erreicht, die zu einem vollständigen Schließen des leeren Werkzeugs führt.

Bei dieser Ausgestaltung werden also der Vorteil der Umlaufsperre einerseits und der freien Betätigung des Werkzeugs andererseits kombiniert. Der Operateur kann das Werkzeug durch Betätigung der Griffteile in beliebiger Reihenfolge stufenlos öffnen und schließen, und trotzdem sieht dieses Instrument die Möglichkeit vor, nach dem Schließen des Werkzeugs dieses in der Schließlage festzulegen und diese Fixierung durch einen Überhub wieder zu lösen.

Dabei ist es günstig, wenn die Teile der Umlaufsperre erst miteinander in Eingriff gelangen, kurz bevor der Betätigungshebel seine an den Griffteil angenäherte Endposition erreicht. Damit steht dem Operateur fast der gesamte Schwenkbereich des Betätigungshebels für die Öffnung und Schließung des Werkzeugs zur Verfügung, erst im letzten Schwenkbereich des Hebels wird die Umlaufsperre angelegt und bei weiterer Betätigung auch wieder gelöst. Der Rastbereich der Umlaufsperre wird bei dieser Ausführung sehr kurz ausgebildet, beispielsweise kann die Umlaufsperre mit nur zwei Rasten auskommen.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines Rohrschaftinstruments mit einem zylindrischen Griffteil und einem einschwenkbaren Handgriff;
- Figur 2:: eine Längsschnittansicht des Griffteils mit eingeschalteter Umlaufsperre vor dem Einschwenken des Griffteils;
- Figur 3:: eine vergrößerte Längsschnittansicht des Griffteils im Bereich des Handgriffs bei vollständig eingeschwenktem Handgriff;
- Figur 4:: eine vergrößerte Teilschnittansicht des rückwärtigen Ende des Handgriffs mit unbetätigtem Handgriff;
- Figur 5:: eine Ansicht ähnlich Figur 4 mit teilweise eingeschwektem Handgriff und wirksamer Rastverbindung;
- Figur 6:: eine Ansicht ähnlich Figur 4 mit dem Handgriff beim Ausschwenken nach dem Lösen der Umlaufsperre durch vollständiges Schließen des Handgriffs;
- Figur 7:: eine Seitenansicht eines abgewandelten Ausführungsbeispiels eines Rohrschaftinstruments;
- Figur 8:: eine Seitenansicht des in Längsrichtung geschnittenen Griffteils des Rohrschaftinstruments der Figur 7 bei gelöster Umlaufsperre;
- Figur 9:: eine Ansicht ähnlich Figur 8 bei Beginn des Eingriffs der Umlaufsperre und
- Figur 10:: eine Ansicht ähnlich Figur 9 mit angelegter Umlaufsperre.

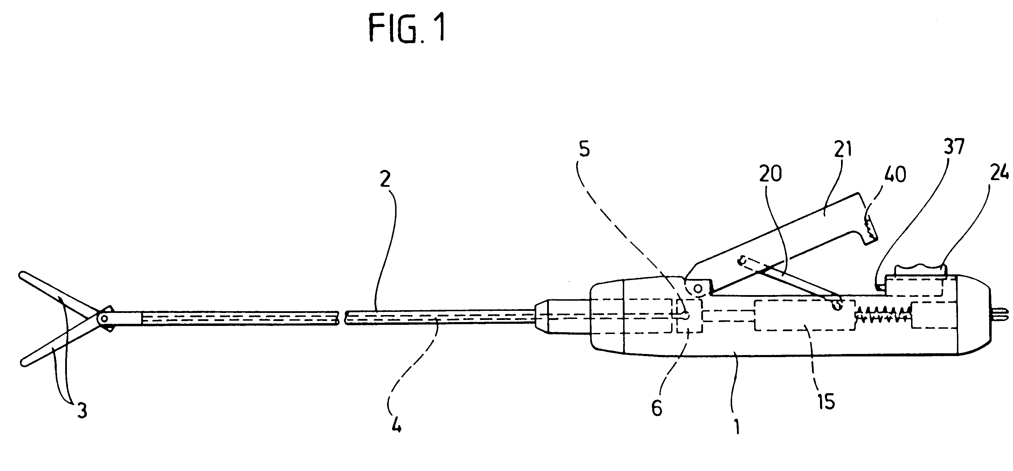

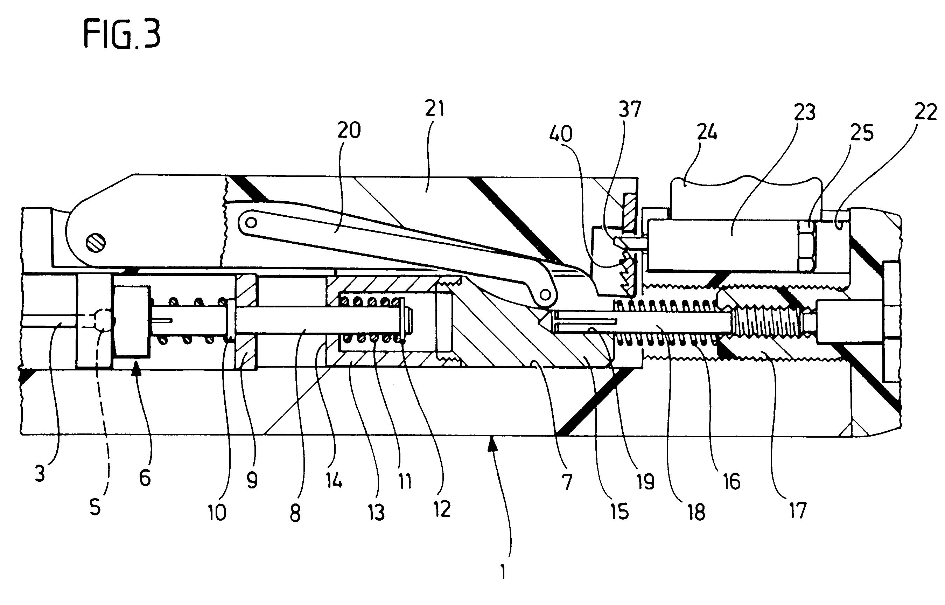

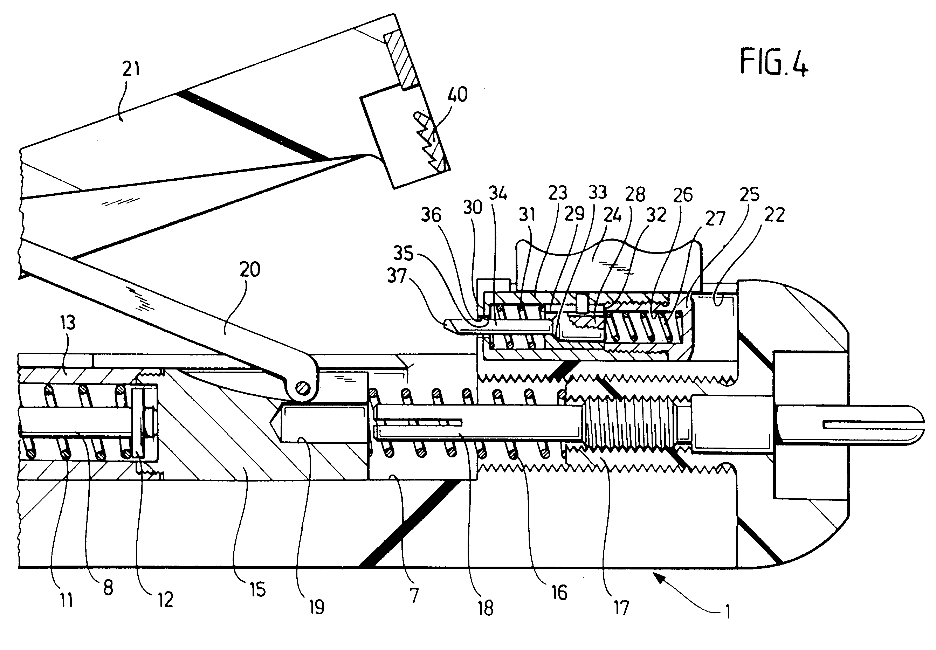

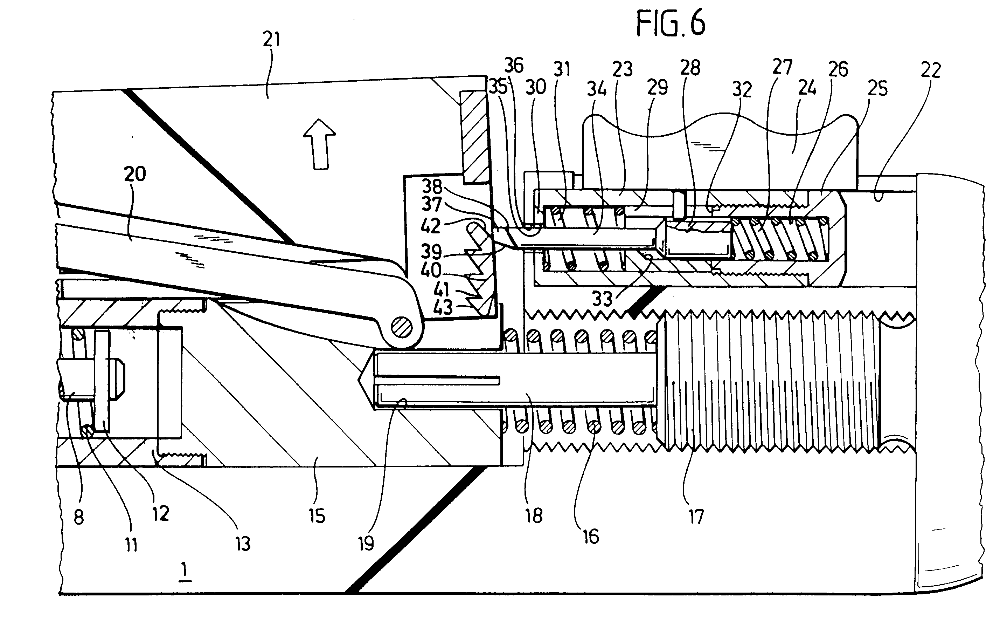

Das in den Figuren 1 bis 6 dargestellte Rohrschaftinstrument umfaßt ein zylindrisches Griffteil 1, an dem ein rohrförmiger Schaft 2 gehalten ist. An dessen Ende sind Werkzeuge 3 beweglich gelagert, die über eine im Inneren des Schafts 2 verschiebbare Schub- und Zugstange 4 betätigbar sind.

Diese Schub- und Zugstange 4 greift mit einem kugeligen Kopf 5 in eine Spannzange 6 ein, die in einer Innenbohrung 7 des Griffteils 1 in Längsrichtung verschieblich gelagert ist. Diese Spannzange 6 weist in der Verlängerung der Schub- und Zugstange 4 eine zylindrische Verlängerung 8 auf, die einen Quersteg 9 in der Innenbohrung 7 durchsetzt und dadurch geführt ist. Der Quersteg 9 bildet einen Anschlag für eine Ringschulter 10, die dadurch die Bewegung der Verlängerung 8 in das Griffteil hinein begrenzt.

Am freien Ende wird die Verlängerung 8 von einer Schraubenfeder 11 umgeben, die sich mit ihrem hinteren Ende an einem Vorsprung 12 der Verlängerung 8 abstützt.

Der von der Schraubenfeder 11 umgebene Teil der Verlängerung 8 taucht in eine Zughülse 13 ein, die mit ihrer vorderen Stirnwand 14 an dem dem Vorsprung 12 abgewandten Ende der Schraubenfeder 11 anliegt und die durch Verschraubung mit einem Gleitstein 15 in der Innenbohrung 7 verbunden ist. An diesem stützt sich eine Druckfeder 16 ab, deren anderes Ende an einem Gewindestopfen 17 anliegt, der die Innenbohrung 7 am rückwärtigen Ende des Griffteils abschließt. Dadurch wird der Gleitstein 15 in der Innenbohrung 7 mit einer in Richtung auf den Schaft 2 weisenden Kraft beaufschlagt.

In den Gewindestopfen 17 ist noch ein Stift 18 eingeschraubt, der in eine Bohrung 19 im Gleitstein 15 eintaucht, wenn der Gleitstein 15 in der Innenbohrung entgegen der Wirkung der Druckfeder 16 verschoben wird. Dieser Stift 18 dient der Herstellung einer elektrischen Verbindung zwischen sich und dem Gleitstein 15 bei zurückgeschobenem Gleitstein, diese Verbindung wird unterbrochen, sobald der Gleitstein 15 in der vorderen Position steht.

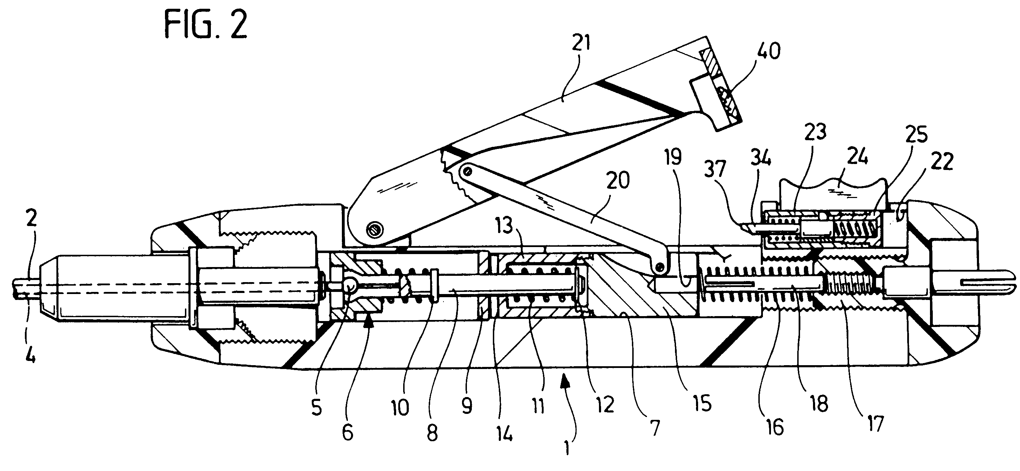

Der Gleitstein 15 ist gelenkig mit einem Arm 20 verbunden, dessen anderes Ende gelenkig an einem Handgriff 21 angelenkt ist, der seinerseits um eine quer zur Längsachse des Griffteils 1 verlaufende Schwenkachse verschwenkbar am Griffteil 1 oberhalb der Innenbohrung 7 gelagert ist. Eine Verschwenkbewegung des Handgriffs 21 wird durch den Arm 20 auf den Gleitstein 15 übertragen, der beim Einschwenken des Handgriffs 21 in der Innenbohrung 7 nach hinten und beim Ausschwenken wieder nach vorne verschoben wird. Durch die am Gleitstein 15 anliegende Druckfeder 16 wird der Handgriff 21 normalerweise in die in Figur 2 dargestellte ausgeschwenkte Stellung verschoben, gegen die Wirkung dieser Druckfeder 16 läßt sich der Handgriff 21 einschwenken.

Im Griffteil befindet sich oberhalb der den Gewindestopfen 17 aufnehmenden Verlängerung der Innenbohrung 7 eine von hinten her zugängliche Bohrung 22, in die von der Rückseite her eine Zylinderbüchse 23 eingesetzt ist. Der Außendurchmesser der Zylinderbüchse 23 entspricht dem Innendurchmesser der Bohrung 22, die Zylinderbüchse 23 ist kürzer ausgebildet als die Bohrung 22, sie kann daher in der Bohrung 22 in Längsrichtung verschoben werden. Zu diesem Zweck ist die Zylinderbüchse 23 mit einem Schieber 24 verbunden, der von der Außenseite des Griffteils l her parallel zur Längsrichtung des Griffteils 1 verschiebbar ist und dabei die Zylinderbüchse 23 mitnimmt. In den jeweiligen Endpositionen wird die Zylinderbüchse 23 dabei in der Bohrung 22 durch eine elastische Raste festgelegt, diese ist in der Zeichnung nicht dargestellt. Die Federkraft dieser Raste ist nicht allzu groß, so daß die Zylinderbüchse 23 relativ leicht aus den jeweiligen Endpositionen heraus verschiebbar ist.

Die Zylinderbüchse 23 ist einseitig offen und auf dieser Seite von einem Gewindestopfen 25 verschlossen, der in einer Sacklochbohrung 26 eine Schraubenfeder 27 aufnimmt.

Diese liegt an einem Kolben 28 an, der seinerseits in einem Hohlkolben 29 verschieblich gelagert ist. Dieser Hohlkolben 29 wiederum ist im Inneren der Zylinderbüchse 23 verschieblich gelagert. Zwischen der dem Gewindestopfen 25 gegenüberliegenden Stirnseite 30 der Zylinderbüchse 23 und dem Hohlkolben 29 ist eine Druckfeder 31 eingelegt, die den Hohlkolben 29 gegen eine Stufe 32 schiebt, die von dem Rand des Gewindestopfens 25 gebildet wird.

Die Einschubtiefe des Kolbens 28 in den Hohlkolben 29 wird durch eine schräge Stufe 33 begrenzt. Mit dem Kolben 28 ist eine Stange 34 verbunden, die die Druckfeder 31 durchsetzt und durch eine Öffnung 35 in der sonst verschlossenen Stirnseite 30 und durch eine Öffnung 36 in der Verlängerung der Bohrung 22 aus dieser austritt; am freien Ende trägt die Stange 34 einen Rastkörper 37 mit einer oberen schräg verlaufenden Aufgleitfläche 38 und einer unteren schräg verlaufenden Aufgleitfläche 39.

Am hinteren Ende des Handgriffs 21 ist in senkrechter Richtung verlaufend eine Raste 40 angeordnet, die auf der der Bohrung 22 abgewandten Seite Rastausnehmungen 41 mit sägezahnförmiger Kontur aufweist. Die Raste 40 ist an einer Seite mit dem Handgriff verbunden, an der Oberseite, an der Unterseite und an der anderen Seite ist die Raste 40 hingegen frei zugänglich. Am oberen Ende weist die Raste 40 auf der den Rastausnehmungen 41 abgewandten Seite eine schräge Aufgleitfläche 42 auf, die etwa parallel zu den schräg verlaufenden Flächen der Rastausnehmungen 41 verläuft. Eine im wesentlichen parallel dazu verlaufende Aufgleitfläche 43 ist auch am unteren Ende der Raste 40 auf der Seite der Rastausnehmungen 41 vorgesehen.

Die Raste 40 wirkt mit dem Rastkörper 37 nach Art einer Umlaufsperre zusammen, dabei bilden der Rastkörper 37, die diesen tragende Stange 34 und der mit der Stange verbundene Kolben 28 ein elastisches Rastelement, das durch die Schraubenfeder 27 einerseits und die Druckfeder 31 andererseits von einer Mittelstellung nach beiden Seiten federnd auslenkbar ist.

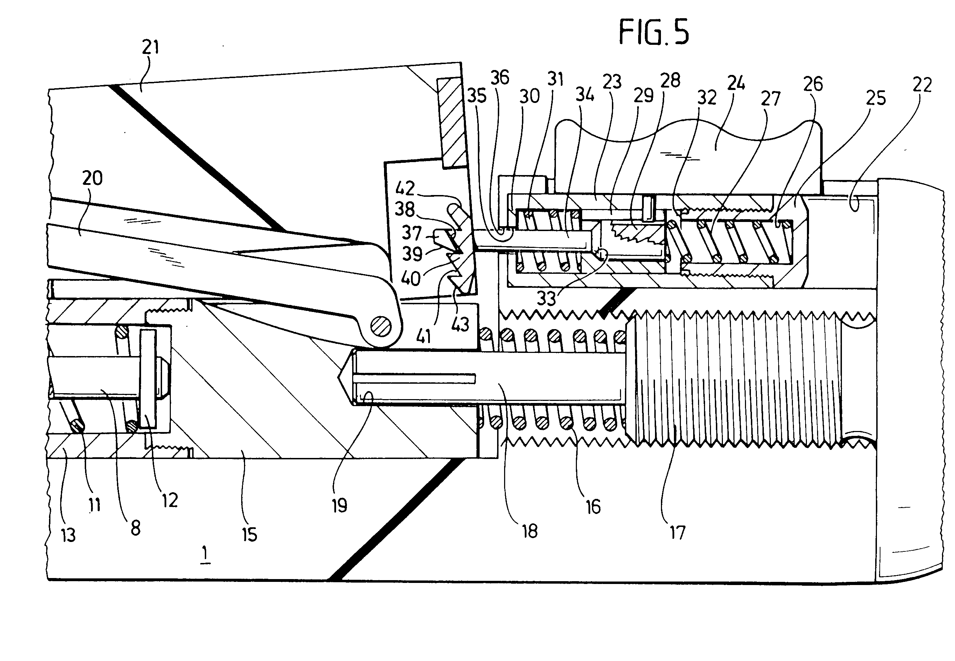

Beim Einschwenken des Handgriffs 21 wird der Gleitstein 15 in der Innenbohrung 7 nach hinten verschoben und nimmt dabei über die Schraubenfeder 11 die Verlängerung 8 mit, diese überträgt diese Bewegung auf die Schub- und Zugstange 4. Bei dieser Schließbewegung gelangen die untere Aufgleitfläche 43 der Raste 40 und die obere Aufgleitfläche 38 des Rastkörpers 37 zur Anlage, und dadurch wird das Rastelement entgegen der Wirkung der Druckfeder 31 verschoben, so daß der Rastkörper 37 über einen Zahn der Raste 40 aufgleiten und danach unter Entspannung der Druckfeder 31 in die erste Rastausnehmung 41 eintreten kann. Beim weiteren Vorschieben des Handgriffs erfolgt der gleiche Vorgang an der schrägen Fläche der Rastausnehmung 41 erneut, bis der Rastkörper 37 in der zweiten Rastausnehmung ruht, wie dies in Figur 5 dargestellt ist. Der Rastkörper 37 verhindert in dieser Position eine Rückbewegung des Handgriffs 21 in die ausgeschwenkte Stellung, so daß der Gleitstein 15 in der einmal erreichten Position in der Innenbohrung 7 fixiert wird.

Bei dieser Rückbewegung des Gleitsteins gelangt schließlich die Ringschulter 10 am Quersteg 9 zur Anlage, so daß eine weitere Verschiebung der Schub- und Zugstange 4 und damit der Spannzange 6 verhindert wird. Trotzdem kann sich der Gleitstein 15 noch weiter nach hinten verschieben lassen, wenn der Handgriff 21 weiter geschlossen wird, da die weitere Bewegung des Gleitsteins gegenüber der Verlängerung 8 der Spannzange 6 durch die Schraubenfeder 11 ermöglicht wird, die bei dieser weiteren Bewegung komprimiert wird. Wenn der Handgriff 21 vollständig geschlossen wird, gelangt der Rastkörper 37 an das obere Ende der Raste 40 und kann nunmehr unter der Wirkung der Druckfeder 31 so weit verschoben werden, daß der Rastkörper 37 etwa oberhalb der Raste 40 in einer durch die Schraubenfeder 27 und die Druckfeder 31 bestimmten Mittellage verbleibt. Schwenkt man jetzt den Handgriff 21 wieder nach außen, gelangt die untere Aufgleitfläche 39 des Rastkörpers 37 an der oberen Aufgleitfläche 42 der Raste 40 zur Anlage und verschiebt dadurch den Rastkörper 37 entgegen der Wirkung der Schraubenfeder 27, das heißt der Kolben 28 wird aus dem Hohlkolben 29 ausgeschoben. Dadurch kann die Raste 40 an dem Rastkörper 37 nach außen vorbeigleiten, der Rastkörper 37 bleibt dabei an der Rückseite der Raste 40 in der Anlage.

Wenn die Raste 40 am Rastkörper 37 vorbeigeschoben worden ist, verschiebt die Schraubenfeder 27 den Rastkörper 37 wieder in die Ausgangslage unterhalb der Raste 40, und beim Schließen des Handgriffs wiederholt sich der beschriebene Vorgang.

Dabei ist wesentlich, daß beim Schließen des Handgriffs die Schub- und Zugstange 4 nur bis zu einer bestimmten Position verschoben werden kann, der danach notwendige Überhub des Handgriffs 21 zur Lösung der Umlaufsperre wird von der Schraubenfeder 11 aufgenommen.

Die beschriebene Funktion der Umlaufsperre setzt voraus, daß sich die Zylinderbüchse 23 immer in der vorgeschobenen Stellung befindet.

Wird die Zylinderbüchse 23 mit Hilfe des Schiebers 24 dagegen in die zurückgezogene Stellung verschoben, ist auch der Rastkörper 37 so weit von der Raste 40 entfernt, daß die Raste 40 unbehindert am Rastkörper 37 vorbeigleiten kann, das heißt der Handgriff kann ohne Wirkung der Raste geöffnet und geschlossen werden. Der Operateur kann allein durch die Stellung des Schiebers 24 bestimmen, ob er eine wirksame Umlaufsperre verwendet oder nicht.

Dabei ist auch möglich, daß die Abstimmung der Federkräfte der Federn 27 und 31 einerseits und der elastischen Rasten, die die Zylinderbüchse 23 in der Bohrung 22 in den Endlagen festlegen, so gewählt wird, daß nach dem Einschalten der Umlaufsperre bei der Rückbewegung des Handgriffs und der dabei auftretenden Rückbewegung des Rastkörpers 37 nicht nur das Rastelement federnd in die Zylinderbüchse 23 eingeschoben wird, sondern eine Verschiebung der Zylinderbüchse 23 in der Bohrung 22 auftritt. Dies führt dazu, daß bei einer nächsten Schließbewegung des Handgriffs der Rastkörper 37 nicht unterhalb der Raste 40 steht und daher nicht in die Rastausnehmungen 41 gelangen kann. Mit anderen Worten schaltet sich die Umlaufsperre nach jedem Umlauf selbsttätig aus. Wenn der Operateur die Umlaufsperre erneut einsetzen will, muß er daher die Zylinderbüchse 23 vor dem Schließen des Handgriffs 21 mit Hilfe des Schiebers 24 in die vorgeschobene Stellung verschieben.

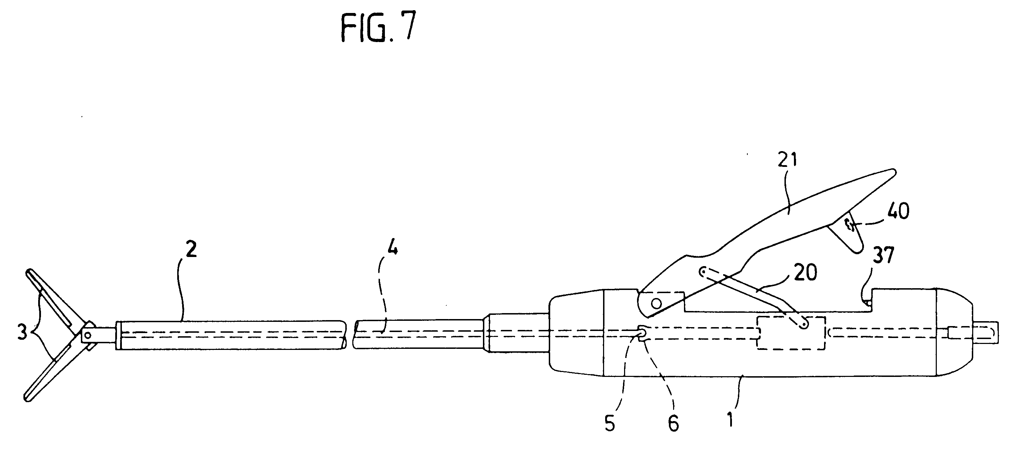

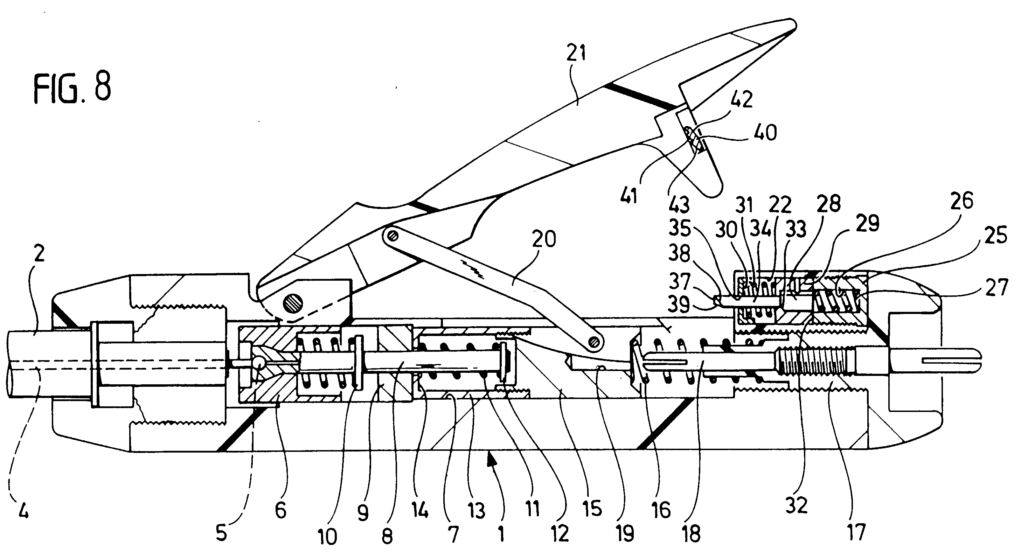

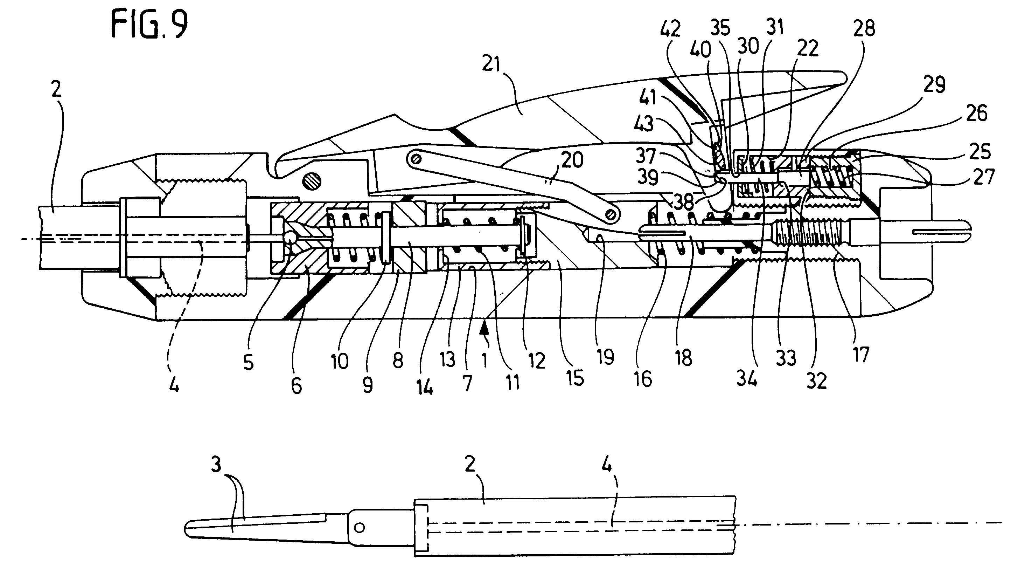

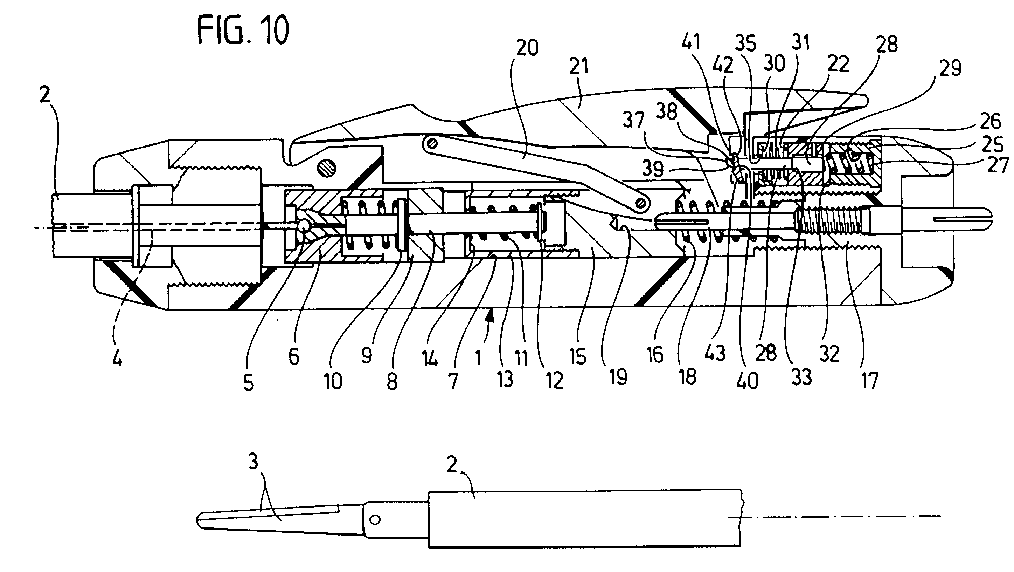

In den Figuren 7 bis 10 ist ein abgewandeltes Ausführungsbeispiel eines Rohrschaftschaftinstrumentes beschrieben. Dieses ist weitgehend gleich aufgebaut wie das Rohrschaftinstrument der Figuren 1 bis 6, einander entsprechende Teile tragen daher dieselben Bezugszeichen. Im Unterschied zum Ausführungsbeispiel der Figuren 1 bis 6 fehlt bei dem Ausführungsbeispiel der Figuren 7 bis 10 die Zylinderbüchse 23, die beim Ausführungsbeispiel der Figuren 1 bis 6 in der Bohrung 22 verschiebbar ist. Bei der Ausführungsform der Figuren 7 bis 10 sind die Teile des Ausführungsbeispiels der Figuren 1 bis 6, die in der Zylinderbüchse 23 gelagert sind, unmittelbar in der Bohrung 22 selbst gelagert, die ihrerseits von den Gewindestopfen 25 verschlossen wird.

Bis auf diesen Unterschied ist der Aufbau im Bereich der Bohrung 22 gleich, es fehlt natürlich auch ein Schieber 24 zur Verschiebung der Zylinderbüchse 23.

Ein weiterer wesentlicher Unterschied gegenüber dem Ausführungsbeispiel der Figuren 1 bis 6 liegt darin, daß die Dimensionierung der Teile der Umlaufsperre so gewählt ist, daß ein vollständiges Schließen des Werkzeugs erfolgt, ehe die untere Aufgleitfläche 43 der Raste 40 und die obere Aufgleitfläche 38 des Rastkörpers 37 sich berühren. Erst wenn die Ringschulter 10 am Quersteg 9 anliegt, so daß eine weitere Verschiebung der Schub- und Zugstange 4 und damit der Spannzange 6 verhindert wird, gelangen bei der weiteren Bewegung des Gleitsteines 15 die untere Aufgleitfläche 43 der Raste 40 und die obere Aufgleitfläche 38 des Rastkörpers 37 zur Anlage, und dadurch wird das Rastelement entgegen der Wirkung der Druckfeder 31 verschoben, so daß der Rastkörper 37 über einen Zahn der Raste 40 aufgleiten und danach unter Entspannung der Druckfeder 31 in die erste Rastausnehmung 41 eintreten kann. Beim weiteren Vorschieben des Handgriffs erfolgt der gleiche Vorgang an der schrägen Fläche der Rastausnehmung 41 erneut, bis der Rastkörper 37 in der zweiten Rastausnehmung ruht. Der Rastkörper 37 verhindert in dieser Position eine Rückbewegung des Handgriffs 21 in die ausgeschwenkte Stellung, so daß der Gleitstein 15 in der einmal erreichten Position in der Innenbohrung 7 fixiert wird.

Dabei ist wesentlich, daß beim Schließen des Handgriffs die Schub- und Zugstange 4 nur bis zu einer bestimmten Position verschoben werden kann. Bis zu dieser Position ist die Umlaufsperre nicht im Eingriff, bis zu dieser Position kann daher die Öffnungs- und Schließbewegung des Werkzeugs jederzeit umgekehrt werden.

Erst nach einer weiteren Verschiebung des Gleitsteines über diese Position hinweg, die durch die Anlage der Ringschulter 10 am Quersteg 9 definiert wird, kann die Umlaufsperre in Eingriff kommen und beim weiteren Schließen des Handgriffs auch wieder gelöst werden.

Dadurch wird es für den Benutzer des Instruments möglich, nach dem Schließen des Werkzeugs wahlweise die Umlaufsperre anzulegen und auch wieder zu lösen, ohne daß das Werkzeug zunächst geöffnet wird. Das Lösen kann nämlich in einfacher Weise dadurch erfolgen, daß der Handgriff 21 vollständig geschlossen wird und dann nur ein wenig wieder geöffnet wird, so daß die Ringschulter 10 noch in der Anlage am Quersteg 9 verbleibt. Falls der Benutzer es wünscht, kann er ausgehend von dieser gelösten Stellung der Umlaufsperre diese wieder verschließen, es ist aber auch möglich, durch weiteres Öffnen des Handgriffs 21 dann das Werkzeug 3 zu öffnen.

## Patentansprüche

1. Chirurgisches Rohrschaftinstrument mit einem Schaft (2) und einer darin verschieblich gelagerten Schub- und Zugstange (4) zur Bewegung eines Werkzeugs (3) am Ende des Schafts und mit einem koaxial zum Schaft angeordneten Griffteil (1) am anderen Ende des Schafts, an dem ein Handgriff (21) um eine quer zur Schaftlängsachse verlaufende Drehachse verschwenkbar gelagert ist, dessen Bewegung über Getriebemittel auf die Schub- und Zugstange (4) übertragbar ist, und mit einem federnden Rastelement (37), das bei der Bewegung des Handgriffs (21) von einer Ausgangsstellung aus an einer Raste (40) vorbeibewegt wird, dabei in diese eingreift und dabei eine Rückbewegung des Handgriffs (21) verhindert, dadurch gekennzeichnet, daß das Rastelement (37) und die Raste (40) eine Umlaufsperre bilden, bei der das Rastelement (37) am Ende des Hubs des Handgriffs (21) an der Raste (40) vollständig vorbeibewegt ist und bei der Rückbewegung an der Rückseite der Raste (40) vorbei in die Ausgangsstellung gelangt.

2. Chirurgisches Rohrschaftinstrument nach Anspruch 1, dadurch gekennzeichnet, daß die Raste (40) am Handgriff (21) gehalten ist.

3. Chirurgisches Rohrschaftinstrument nach Anspruch 2, dadurch gekennzeichnet, daß die Raste (40) am freien Ende des Handgriffs (21) senkrecht zur Längsausdehnung des Handgriffs (21) angeordnet ist.

4. Chirurgisches Rohrschaftinstrument nach einem der voranstehenden Ansprüche, dadurch gekennzeichnet, daß das federnde Rastelement (37) im Griffteil (1) im wesentlichen parallel zum Schaft (2) verschieblich gelagert ist.

5. Chirurgisches Rohrschaftinstrument nach einem der voranstehenden Ansprüche, dadurch gekennzeichnet, daß das Rastelement (37) mittels eines Betätigungselements (24) zwischen zwei Stellungen verschiebbar ist, nämlich einer vorgeschobenen Rastposition, in der das Rastelement (37) mit der Raste (40) als Umlaufsperre wirkt, und einer zurückgezogenen Freilaufposition, in der das Rastelement (37) von der Raste (40) dauerhaft entfernt ist.

6. Chirurgisches Rohrschaftinstrument nach einem der voranstehenden Ansprüche, dadurch gekennzeichnet, daß das federnde Rastelement (37) am Griffteil (1) von einer Mittelstellung aus in beiden Bewegungsrichtungen gegen die Wirkungen einer Feder (27, 31) elastisch verschiebbar ist.

7. Chirurgisches Rohrschaftinstrument nach Anspruch 6, dadurch gekennzeichnet, daß die Federkraft, durch die das Rastelement (37) in die Raste (40) gedrückt wird, größer ist als die Federkraft, die das Rastelement gegen die Rückseite der Raste (40) andrückt.

8. Chirurgisches Rohrschaftinstrument nach Anspruch 6 oder 7, dadurch gekennzeichnet, daß das Rastelement (37) an einem Kolben (28) gehalten ist, der von einer ersten Feder (27) beaufschlagt bis an einen Anschlag (33) in einen Hohlkolben (29) einschiebbar ist, und daß der Hohlkolben (29) von einer zweiten Feder (31) in entgegengesetzter Richtung gegen einen festen Anschlag (32) einer Führung (23) des Hohlkolbens (29) beaufschlagt wird.

9. Chirurgisches Rohrschaftinstrument nach Anspruch 4 und 8, dadurch gekennzeichnet, daß die Führung eine Zylinderhülse (23) ist, die mittels des Betätigungselements (24) im Griffteil (1) verschiebbar ist.

10. Chirurgisches Rohrschaftinstrument nach Anspruch 9, dadurch gekennzeichnet, daß die Zylinderhülse (23) in einer Bohrung (22) des Griffteils (1) verschiebbar ist.

11. Chirurgisches Rohrschaftinstrument nach Anspruch 10, dadurch gekennzeichnet, daß die Zylinderhülse (23) in der Bohrung (22) in den beiden Endstellungen durch elastische Rasten gehalten ist.

12. Chirurgisches Rohrschaftinstrument nach einem der voranstehenden Ansprüche, dadurch gekennzeichnet, daß die Getriebemittel eine Federverbindung (11, 12, 13) umfassen, die nach dem Erreichen einer Endposition der Schub- und Zugstange (4) eine weitere Schließbewegung des Handgriffs (21) ermöglicht.

13. Chirurgisches Rohrschaftinstrument nach Anspruch 12, dadurch gekennzeichnet, daß die Endposition der Schub- und Zugstange (4) durch einen Anschlag (9) definiert wird, an dem ein Teil der Schub- und Zugstange (10) zur Anlage kommt.

14. Chirurgisches Rohrschaftinstrument nach einem der Ansprüche 12 oder 13, dadurch gekennzeichnet, daß die Federverbindung durch eine die Schub- und Zugstange umgebende, sich einseitig an einem Vorsprung (12) derselben abstützende Druckfeder (11) gebildet wird, an deren anderem Ende eine die Druckfeder (11) umgebende Zughülse (13) angreift.

15. Chirurgisches Rohrschaftinstrument nach einem der voranstehenden Ansprüche, dadurch gekennzeichnet, daß die Raste (40) und das Rastelement (37) erst in Eingriff gelangen, wenn das Betätigungselement (21) eine Position erreicht hat, die zu einem vollständigen Schließen des leeren Werkzeugs (3) führt.

16. Chirurgisches Rohrschaftinstrument nach Anspruch 15, dadurch gekennzeichnet, daß die Raste (40) und das Rastelement (37) erst miteinander in Eingriff gelangen kurz bevor der Betätigungshebel seine an den Griffteil (1) angenäherte Endposition erreicht.

## Claims

1. A tubular-shaft surgical instrument comprising a shaft (2) and a push-pull rod (4) displaceably mounted therein for moving an implement (3) at the end of the shaft, and comprising a gripping member (1) arranged co-axially with the shaft at the other end thereof, a handle (21) being mounted on the gripping member (1) so as to pivot about an axis of rotation extending transversely to the longitudinal axis of the shaft, the movement of the handle (21) being transmittable to the push-pull rod (4) via transmission means, and comprising a resilient catching member (37) which, during movement of the handle (21), is moved out of a starting position past a detent (40), engages therein and thus prevents a return movement of the handle (21), characterised in that the catching member (37) and the detent (40) form a circulating catch, wherein the catching member (37) has moved completely past the detent (40) at the end of the travel of the handle (21) and, during the return movement, moves past the rear of the detent (40) into the starting position.

2. A tubular-shaft surgical instrument according to claim 1, characterised in that the detent (40) is held on the handle (21).

3. A tubular-shaft surgical instrument according to claim 2, characterised in that the detent (40) is arranged on the free end of the handle (21) perpendicularly to the longitudinal extent of the handle (21).

4. A tubular-shaft surgical instrument according to any one of the preceding claims, characterised in that the resilient catching member (37) is mounted in the gripping member (1) so as to be displaceable substantially parallel to the shaft (2).

5. A tubular-shaft surgical instrument according to any one of the preceding claims, characterised in that the catching member (37) is displaceable between two positions by means of an actuating member (24), namely an advanced catching position in which the catching member (37) acts as a circulating catch together with the detent (40), and a retracted neutral position in which the catching member (37) is permanently removed from the detent (40).

6. A tubular-shaft surgical instrument according to any one of the preceding claims, characterised in that the resilient catching member (37) is resiliently displaceable on the gripping member (1) out of a centre position in both directions of movement against the action of a spring (27, 31).

7. A tubular-shaft surgical instrument according to claim 6, characterised in that the spring force, by means of which the catching member (37) is pushed into the detent (40), is greater than the spring force pushing the catching member towards the rear of the detent (40).

8. A tubular-shaft surgical instrument according to claim 6 or 7, characterised in that the catching member (37) is held on a piston (28) acted upon by a first spring (27) and pushable into a hollow piston (29) as far as a stop (33), and in that the hollow piston (29) is acted upon by a second spring (31) in the opposite direction towards a fixed stop (32) of a guide (23) of the hollow piston (29).

9. A tubular-shaft surgical instrument according to claims 4 and 8, characterised in that the guide is a cylindrical sleeve (23) displaceable in the gripping member (1) by means of the actuating member (24).

10. A tubular-shaft surgical instrument according to claim 9, characterised in that the cylindrical sleeve (23) is displaceable in a bore (22) of the gripping member (1).

11. A tubular-shaft surgical instrument according to claim 10, characterised in that the cylindrical sleeve (23) is held in the bore (22) in the two end positions by resilient detents.

12. A tubular-shaft surgical instrument according to any one of the preceding claims, characterised in that the transmission means comprise a spring connection (11, 12, 13) making a further closing movement of the handle (21) possible when the push-pull rod (4) has reached an end position.

13. A tubular-shaft surgical instrument according to claim 12, characterised in that the end position of the push-pull rod (4) is defined by a stop (9), against which a part (10) of the push-pull rod comes to rest.

14. A tubular-shaft surgical instrument according to either one of claims 12 and 13, characterised in that the spring connection is formed by a compression spring (11) surrounding the push-pull rod and supported at one end against a projection (12) of the push-pull rod, a tension sleeve (13), surrounding the compression spring (11), engaging the other end thereof.

15. A tubular-shaft surgical instrument according to any one of the preceding claims, characterised in that the detent (40) and the catching member (37) do not engage until the operating member (21) has reached a position resulting in complete closure of the empty implement (3).

16. A tubular-shaft surgical instrument according to claim 15, characterised in that the detent (40) and the catching member (37) do not mutually engage until shortly before the operating lever reaches its end position moved towards the gripping member (1).

## Revendications

1. Instrument chirurgical à fût tubulaire comportant un fût (2) et une tige de poussée et de traction (4) montée coulissante dans celui-ci pour déplacer un outil (3) à l'extrémité du fût, et un élément de préhension (1) agencé coaxialement par rapport au fût sur l'autre extrémité du fût, sur laquelle une poignée (21) est montée à pivotement autour d'un axe de rotation s'étendant transversalement à l'axe longitudinal du fût, dont le mouvement est susceptible d'être transmis à la tige de poussée et de traction (4) par l'intermédiaire de moyens de transmission, et comportant un élément d'encliquetage (37) élastique qui, lors du mouvement de la poignée (21), est déplacé depuis une position initiale devant un cliquet (40), s'engage dans celui-ci et empêche en ce faisant un mouvement de retour de la poignée (21), caractérisé en ce que l'élément d'encliquetage (37) et le cliquet (40) forment un blocage de rotation lors duquel, à la fin de la course de la poignée (21), l'élément d'encliquetage (37) est déplacé complètement devant le cliquet (40) et parvient au cours du mouvement de retour dans la position initiale en passant devant la face postérieure du cliquet (40).

2. Instrument chirurgical à fût tubulaire selon la revendication 1, caractérisé en ce que le cliquet (40) est maintenu sur la poignée (21).

3. Instrument chirurgical à fût tubulaire selon la revendication 2, caractérisé en ce que le cliquet (40) est agencé sur l'extrémité libre de la poignée (21) perpendiculairement à l'extension longitudinale de la poignée (21).

4. Instrument chirurgical à fût tubulaire selon l'une quelconque des revendications précédentes, caractérisé en ce que l'élément d'encliquetage (37) élastique est monté sensiblement parallèle au fût (2), à déplacement dans l'élément de préhension (1).

5. Instrument chirurgical à fût tubulaire selon l'une quelconque des revendications précédentes, caractérisé en ce que l'élément d'encliquetage (37) est susceptible d'être déplacé au moyen d'un élément d'actionnement (24) entre deux positions, à savoir une position d'encliquetage avancée, dans laquelle l'élément d'encliquetage (37) agit comme blocage de rotation avec le cliquet (40), et une position de course libre en retrait dans laquelle l'élément d'encliquetage (37) est éloigné du cliquet (40) de manière durable.

6. Instrument chirurgical à fût tubulaire selon l'une quelconque des revendications précédentes, caractérisé en ce que l'élément d'encliquetage (37) élastique est susceptible d'être déplacé dans les deux directions de déplacement de manière élastique sur l'élément de préhension (1) depuis une position médiane à l'encontre d'un ressort (27, 31).

7. Instrument chirurgical selon la revendication 6, caractérisé en ce que la force de ressort par laquelle l'élément d'encliquetage (37) est pressé dans le cliquet (40) est plus grande que la force de ressort qui pousse l'élément d'encliquetage contre la face postérieure du cliquet (40).

8. Instrument chirurgical selon l'une ou l'autres des revendications 6 et 7, caractérisé en ce que l'élément d'encliquetage (37) est maintenu sur un piston (28) qui, sollicité par un premier ressort (27), est susceptible d'être enfiché dans un piston creux (29) jusqu'à une butée (33), et en ce que le piston creux (29) est sollicité par un second ressort (31) en direction opposée contre une butée fixe (32) d'un guidage (23) du piston creux (29).

9. Instrument chirurgical selon les revendications 4 et 8, caractérisé en ce que le guidage est une douille cylindrique (23) qui est susceptible d'être déplacée dans l'élément de préhension (1) au moyen de l'élément d'actionnement (24).

10. Instrument chirurgical selon la revendication 9, caractérisé en ce que la douille cylindrique (23) est susceptible d'être déplacée dans un perçage (22) de l'élément de préhension (1).

11. Instrument chirurgical selon la revendication 10, caractérisé en ce que la douille cylindrique (23) est maintenue dans le perçage (22) dans les deux positions finales par des cliquets élastiques.

12. Instrument chirurgical selon l'une quelconque des revendications précédentes, caractérisé en ce que les moyens de transmission comprennent une liaison élastique (11, 12, 13) qui, après avoir atteint une position finale de la tige de poussée et de traction (4), permet un autre mouvement de fermeture de la poignée (21).

13. Instrument chirurgical selon la revendication 12, caractérisé en ce que la position finale de la tige de poussée et de traction (4) est définie par une butée (9) sur laquelle une partie de la tige de poussée et de traction (10) vient en appui.

14. Instrument chirurgical selon l'une ou l'autre des revendications 12 et 13, caractérisé en ce que la liaison par ressort est formée par un ressort de compression (11) qui entoure la tige de poussée et de traction et prend appui d'un côté sur une saillie (12) de ladite tige, à l'autre extrémité duquel attaque une douille de traction (13) entourant le ressort de compression.

15. Instrument chirurgical selon l'une quelconque des revendications précédentes, caractérisé en ce que le cliquet (40) et l'élément d'encliquetage (37) parviennent seulement en engagement lorsque l'élément d'actionnement (24) a atteint une position qui mène à la fermeture complète de l'outil (3) vide.

16. Instrument chirurgical selon la revendication 15, caractérisé en ce que le cliquet (40) et l'élément d'encliquetage (37) parviennent seulement en engagement mutuel juste avant que le levier d'actionnement ait atteint sa position finale rapprochée de l'élément de préhension (1).
